# EUROPEAN PATENT APPLICATION

(11) **EP 4 786 784 A1**
(43) Date of publication of application: **05.08.2026**
(21) Application number: 26155156.8
(22) Date of filing: 29.01.2026
(51) Int. Cl.: F04B 43/00, F04B 43/12, F04B 49/06, F04B 51/00, A61M 5/142

(54) **SYSTEM AND METHOD FOR DETERMINING MALFUNCTION IN A FLUID CIRCUIT FOR BLOOD PROCESSING**

(30) Priority: 30.01.2025 US 202563751626 P
(71) Applicant: Fenwal, Inc., Lake Zurich, IL 60047 (US)
(72) Inventor: MAHER, Jeffrey R., Lake Zurich, 60047 (US); QUEZADA, Francesca S., Lake Zurich, 60047 (US); DICOLA, Nicholas R., Lake Zurich, 60047 (US)
(74) Representative: Maikowski & Ninnemann Patentanwälte Partnerschaft mbB

(57) **Abstract**

System and method for identifying a malfunction in a peristaltic pump (110) which comprises a rotor (150) with at least one roller (140), the method comprising the steps: obtaining a first upstream pressure value at a first rotor position in which the roller is positioned away from the tubing (120); activating the peristaltic pump until the rotor reaches a second position in which the roller has completed at least one rotation; obtaining a second upstream pressure value when the rotor is in the second position; determining the pressure difference between the first and the second upstream values; and generating an alert if the determination indicates pump operation abnormality. Alternatively, the method may also identify pump malfunction by determining the pressure difference from downstream pressure values.

## Description

### TECHNICAL FIELD

The present disclosure relates to detecting malfunctions in a tubing kit and/or a defect in a peristaltic pump used in a system for pumping and distributing fluids. More particularly, the present disclosure is directed to determining the type and location of such malfunctions and/or defects.

### BACKGROUND

Peristaltic pumps are commonly used in applications, such as medical and, more particularly, blood processing applications, where a disposable fluid circuit including flexible tubing is used to move fluid through the circuit. The tubing of the fluid circuit conveys fluid throughout the fluid circuit. A portion of the tubing is associated with a peristaltic pump. The peristaltic pump includes a rotating pump head or roller that, upon activation, compresses and releases tubing that is mounted onto and looped about the pump head. An example of a peristaltic pump and its use in the field of biological fluid processing is described in U.S. Patent No. 5,484,239, the contents of which are incorporated herein by reference.

In some cases, the movement of fluid through the circuit may be compromised due to leaks in the tubing of the circuit or a malfunctioning pump. Leaks make it difficult or impossible to pressurize fluids and effectively move the circuit, In other cases, the pump head may malfunction due to wear and tear over time or breakage, resulting in the roller(s) not properly occluding the tubing loop.

In accordance with the operation of a peristaltic pump, by occluding the tubing's internal lumen by compression, a pressure gradient is created, allowing for continuous liquid flow. The decompression of the tubing creates a constant vacuum on the suction side of the pump. After being decompressed, the tubing returns to its initial shape before the next compression cycle begins. This repeated compression and decompression subjects the tubing to a fatigue phenomenon, caused by cyclic loading with stresses that are typically lower than the material's rupture stress and within its elastic domain (allowing for reversible deformation). Over time, localized damage accumulates, leading to the formation of cracks. Once a crack reaches a critical size, it can rapidly propagate, ultimately causing material fracture. Defects/failures in the materials may also be the result of pump misloading or manufacturing defects in the tubing loop.

Thus, it would be desirable to provide a system and method capable of identifying leakage or other pump malfunctions in advance, allowing prompt action, such as replacing tubing or fluid circuit, repairing or replacing a pump head, to be taken before a system failure occurs.

### SUMMARY

In an aspect, the present disclosure is directed to a system for pumping and distributing fluid including flexible tubing defining a fluid path for conveying biological or medical fluid from a source for processing. The system includes an external peristaltic pumping unit associated with the flexible tubing, wherein the peristaltic pumping unit includes a pump race for receiving the tubing, a pump rotor carrying at least one roller, and a drive unit for rotating the rotor and thereby rotating the at least one roller within the pump race wherein the flexible tubing includes an upstream region positioned before the roller and a downstream region positioned after the roller; at least one pressure sensor in communication with the fluid path, wherein the at least one pressure sensor includes an upstream pressure sensor configured to detect at least one pressure value of the fluid passing through the upstream region. The system includes a controller configured to operate the peristaltic pumping unit in a diagnostic mode, in which the rotor is rotated with the at least one roller initially positioned away from the tubing at time t=0; and the drive unit drives the at least one roller to engage the tubing after a time interval Δt, thereby compressing the tubing to measure the at least one pressure value. The controller is further configured to receive from the upstream pressure sensor a first upstream pressure value of the at least one pressure value of the pressure in the fluid passing through the upstream region at time t=0; determine a difference between the first upstream pressure value and a second upstream pressure value, wherein the second upstream pressure value corresponds to the pressure in the fluid passing through the upstream region obtained from the upstream pressure sensor after the at least one roller completes at least one rotation. The controller is configured to generate an upstream alert if the determination indicates pump operation abnormality.

In another aspect, the present disclosure is directed to a system for pumping and distributing fluid includes flexible tubing defining a fluid path for conveying biological or medical fluid from a source for processing. The system includes an external peristaltic pumping unit associated with the flexible tubing, said peristaltic pumping unit including a pump race for receiving the tubing, a pump rotor carrying at least one roller, and a drive unit for rotating the rotor and thereby rotating the at least one roller within the pump race wherein the flexible tubing includes an upstream region positioned before the roller and a downstream region positioned after the roller; at least one pressure sensor in communication with the fluid path, wherein the at least one pressure sensor includes a downstream pressure sensor configured to detect at least one pressure value of the fluid passing through the downstream region; a controller configured to operate the peristaltic pumping unit in a diagnostic mode, in which the rotor is rotated with the at least one roller initially positioned away from the tubing at time t=0; and the drive unit drives the at least one roller to engage the tubing after a time interval Δt, thereby compressing the tubing to measure the at least one pressure value, wherein the controller is further configured to receive from the downstream pressure sensor a first downstream pressure value of the at least one pressure value of the pressure in the fluid passing through the downstream region at time t=0; determine a difference between the first downstream pressure value and a second downstream pressure value, wherein the second downstream pressure value corresponds to the pressure in the fluid passing through the downstream region obtained from the downstream pressure sensor after the at least one roller completes at least one rotation; and generate a downstream alert if the determination indicates pump operation abnormality.

In yet another aspect, the present disclosure is directed to a method for determining a malfunction in a tubing kit mounted on a peristaltic pump unit for pumping and distributing fluid, the peristaltic pumping unit comprising at least one roller, a flexible tubing having an upstream region positioned before the at least one roller where fluid enters the pump and a downstream region positioned after the at least one roller where fluid exits the pump, a race configured to receive the tubing, and at least one pressure sensor, the method comprising steps: (i) placing a tubing in flow communication with fluid source onto the peristaltic pumping element included in the system; (ii) activating the peristaltic pumping unit with a diagnostic mode to allow the at least one roller moving to a retracted position in which the at least one roller is positioned away from the race; (iii) activating the peristaltic pumping unit to allow for fluid flowing from the fluid source to the pumping unit, the at least one roller moving to an engaged position in which the at least one roller engages with the race and rotating the at least one roller within the pump race; (iv) obtaining from an upstream pressure sensor included in the at least one pressure sensor a first upstream pressure value corresponding to the pressure in the fluid passing through an upstream region at time t=0; (v) allowing the at least one roller to complete at least one rotation, wherein the at least one roller moving to the retracted position after completing the at least one rotation; (vi) obtaining from the upstream pressure sensor a second upstream pressure value corresponding to the pressure in the fluid passing through the upstream region after step (v); (vii) determining an upstream pressure difference between the first upstream pressure value and the second upstream pressure value; (viii) generating a first alert if the upstream pressure difference exceeds or falls below a preset threshold range, wherein the preset threshold range corresponding to an expected upstream pressure gradient between upstream pressure values measured before and after the at least one roller completes a rotation; or (ix) generating a second alert if the upstream pressure difference is zero or nearly absent.

In yet another aspect, the present disclosure is directed to a method for determining a malfunction in a tubing kit mounted on a peristaltic pump unit for pumping and distributing fluid, the peristaltic pumping unit comprising at least one roller, a flexible tubing having an upstream region positioned before at least one roller where fluid enters the pump and a downstream region positioned after at least one roller where fluid exits the pump, a race configured to receive the tubing, and at least one pressure sensor, the method comprising steps: (i) placing a tubing in flow communication with fluid source onto the peristaltic pumping element included in the system; (ii) activating the peristaltic pumping unit with a diagnostic mode to allow at least one roller moving to a retracted position in which at least one roller is positioned away from the race; (iii) activating the peristaltic pumping unit to allow for fluid flowing from the fluid source to the pumping unit, the at least one roller moving to an engaged position in which the at least one roller engages with the race and rotating the at least one roller within the pump race; (iv) obtaining from a downstream pressure sensor included in the at least one pressure sensor a first downstream pressure value corresponding to the pressure in the fluid passing through the downstream region at time t=0; (v) allowing the at least one roller to complete at least one rotation, wherein the at least one roller moving to the retracted position after completing the at least one rotation; (vi) obtaining from the downstream pressure sensor a second upstream pressure value corresponding to the pressure in the fluid passing through the downstream region after step (v); (vii) determining a downstream pressure difference between the first downstream pressure value and the second downstream pressure value; (viii) generating a first alert if the downstream pressure difference exceeds or falls below a preset threshold range, wherein the preset threshold range corresponding to an expected downstream pressure gradient between downstream pressure values measured before and after the at least one roller completes a rotation; or (ix) generating a second alert if the downstream pressure difference is zero or nearly absent.

In yet another aspect, the present disclosure is directed to a method for identifying a pump head failure in the system for pumping and distributing fluid including peristaltic pumping element comprising at least one roller, a flexible tubing having an upstream region positioned before the at least one roller where fluid enters the pump and a downstream region positioned after the at least one roller where fluid exits the pump, a race configured to receive the tubing, and at least one pressure sensor, wherein the method comprising steps: (i) placing a tubing in flow communication with fluid source onto the peristaltic pumping element included in the system; (ii) activating the peristaltic pumping element with a diagnostic mode to allow the at least one roller moving to a retracted position in which the at least one roller is positioned away from the race; (iii) activating the peristaltic pumping element to allow for fluid flowing from the fluid source to the pumping unit, the at least one roller moving to an extended position in which the at least one roller engages with the race and rotating the at least one roller within the pump race ; (iv) obtaining from a upstream pressure sensor included in the at least one pressure sensor a first upstream pressure value corresponding to the pressure in the fluid passing through the upstream region at time t=0; (v) allowing the at least one roller to complete at least one rotation in the diagnostic mode, wherein the at least one roller moving to the retracted position after completing the at least one rotation; (vi) obtaining from the upstream pressure sensor a second upstream pressure value corresponding to the pressure in the fluid passing through the upstream region after step (v); (vii) determining an upstream pressure difference between the first upstream pressure value and the second upstream pressure value; (viii) generating a pump head failure alert if the upstream pressure difference is zero or nearly absent..

In yet another aspect, the present disclosure is directed to a method for identifying a pump head failure in the system for pumping and distributing fluid including peristaltic pumping element comprising at least one roller, a flexible tubing having an upstream region positioned before at least one roller where fluid enters the pump and a downstream region positioned after at least one roller where fluid exits the pump, a race configured to receive the tubing, and at least one pressure sensor, wherein the method comprising steps: (i) placing a tubing in flow communication with fluid source onto the peristaltic pumping element included in the system; (ii) activating the peristaltic pumping element with a diagnostic mode to allow at least one roller moving to a retracted position in which at least one roller is positioned away from the race; (iii) activating the peristaltic pumping element to allow for fluid flowing from the fluid source to the pumping unit, at least one roller moving to an extended position in which at least one roller engages with the race and rotating at least one roller within the pump race; (iv) obtaining from a downstream pressure sensor included in the at least one pressure sensor a first downstream pressure value corresponding to the pressure in the fluid passing through the downstream region at time t=0; (v) allowing at least one roller to complete at least one rotation in the diagnostic mode; (vi) obtaining from the downstream pressure sensor a second downstream pressure value corresponding to the pressure in the fluid passing through the downstream region after step (v); (vii) determining the downstream pressure difference between the first downstream pressure value and the second downstream pressure value; (viii) generating a pump head failure alert if the downstream pressure difference is zero or nearly absent.

### DESCRIPTION OF THE DRAWINGS

Fig. 1 illustrates an exemplary system for pumping and distributing fluid with fluid flowing in one direction.
Fig. 2 illustrates an exemplary step of the system for pumping and distributing fluid of Fig. 1 with fluid flowing in an opposite direction.

### DETAILED DESCRIPTION

A more detailed description of the systems and methods in accordance with the present disclosure is set forth below. It should be understood that the description below of specific devices and methods is intended to be exemplary, and not exhaustive of all possible variations or applications. Thus, the scope of the disclosure is not intended to be limiting and should be understood to encompass variations or embodiments that would be apparent to persons of ordinary skill.

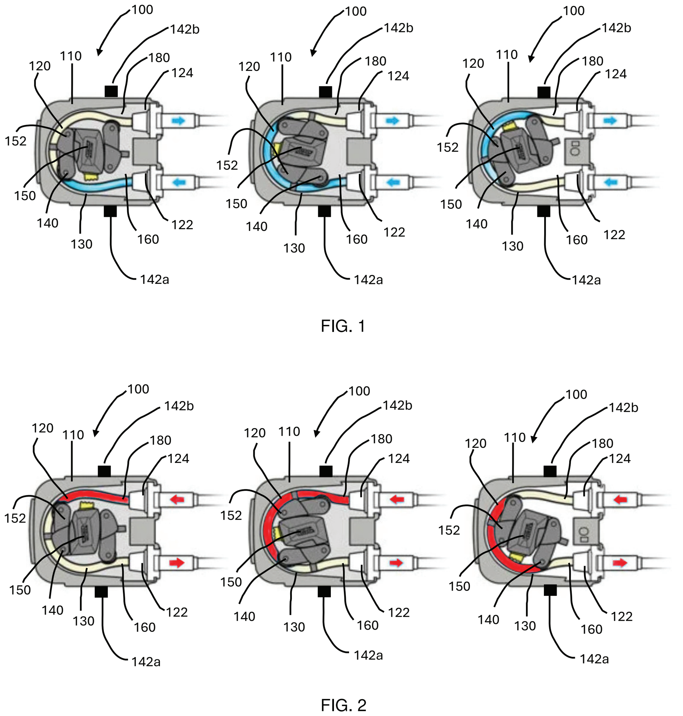

Shown in Fig. 1 is an embodiment of a system 100 for pumping and distributing fluid. In this exemplary system 100, it includes a flexible, deformable tubing 120 defining a fluid path configured to convey biological or medical fluids from a source to a designated processing area, wherein the tubing 120 is made of a biocompatible, sterilizable material suitable for medical or laboratory use. The deformable tube 120 may be made from polymeric materials such as polyvinyl chloride (PVC), silicone or thermoplastic elastomers, suitable for repeated compression and relaxation. The fluid path is adapted to maintain fluid integrity and prevent contamination while enabling precise control of fluid movement, allowing for safe and efficient transport of sensitive biological materials such as blood, saline, medications, or diagnostic samples from a storage source to the intended processing or delivery location.

The system 100 further includes an external peristaltic pumping unit 110 operatively associated with the flexible tubing 120. The pumping unit 110 comprises a pump race 130 configured to securely receive and hold the flexible tubing 120 in place, allowing the tubing 120 to be compressed and released by the at least one roller 140 without slippage or misalignment. The system 100 includes a pump rotor 152 mounted within the pump race 130 and configured to carry the at least one roller 140, where the rotor's rotation causes the roller(s) to alternately engage and disengage the tubing 120, creating controlled fluid displacement along the fluid path. The system 100 may also include a drive unit 150 operatively connected to the pump rotor 152, providing rotational force to the rotor 152 to enable the continuous, uniform rotation of the at least one roller 140 within the pump race 130. This rotational action of the drive unit 150 allows for the precise regulation of fluid flow by rhythmically compressing and releasing the tubing 120, generating a peristaltic motion that propels the fluid through the tubing 120 from an inlet 122 to an outlet 124.

In the exemplified system 100, the at least one roller 140 is configured to engage with the flexible tubing 120, where the roller 140 is shaped and positioned to effectively compress the tubing 120 and control fluid flow. The roller 140 moves between two positions: an engaged position and a retracted position. When the roller 140 moves to the engaged position, it presses against the flexible tubing 120 to compress and occlude it fully, thereby temporarily blocking or reducing fluid flow through the tubing 120. This compression creates a positive pressure downstream 180 of the roller 140, allowing for controlled movement of fluid along the tubing 120. When the roller 140 moves to the retracted position, the roller 140 disengages from the tubing 120, allowing the tubing 120 to expand back to its original shape and enabling fluid to enter the pump 110 from an upstream area 160 of the roller 140.

The drive unit 150 is operatively connected to the at least one roller 140 and configured to control its movement with precision. By moving the roller 140 between the engaged and retracted positions, the drive unit 150 creates a peristaltic pumping action that sequentially compresses and releases the tubing 120 along its path, propelling the fluid forward in a controlled, pulsatile flow.

The tube is positioned along the pump race 130 where the at least one roller 140 can engage and disengage with it. The upstream region 160 is referred as the section of fluid circuit positioned before the roller 140, where fluid enters the pump 110. A downstream region 180 is referred as the section of tubing 120 located after the roller 140, where fluid exits the pump 110. When the roller 140 moves to the engaged (extended) position, it engages with the flexible tubing 120 compressing and partially or fully closing off the tube at that point. This compression decreases the interval volume of the tubing 120 in the region where the roller 140 is engaged, which forces the fluid ahead of the roller 140 to move forward. This reduced volume effectively increases the internal pressure within the compressed section of the tube. With the tube compressed, a positive pressure is created in the downstream region 180 propelling the fluid forward, overcoming any resistance in the downstream 180 tubing and moving it toward the pump's outlet 124. In other words, the roller 140's compression displaces fluid through the tubing 120 by creating this positive pressure. This positive pressure continues as long as the roller 140 remains in the engaged (extended) position, maintaining forward fluid flow until the roller 140 moves back to the retracted position. When the roller 140 is in the retracted position, it creates a negative pressure in the upstream region 160 producing a suction effect to pull the fluid into the tubing 120. After the roller 140 moves to the retracted position, the tubing 120 expands, creating suction to draw in more fluid, which is then subsequently pushed forward in the next engaged (extended) position, thus maintaining a continuous flow. The pressure gradient develops within the tubing 120 in mainly the upstream region 160 and downstream region 180 due to the sequential compression and expansion of the tubing 120 by the roller 140 as it completes a rotation. As described in more detail below, the absence of such pressure gradient would potentially demonstrate issues such as leakage in the tubing 120 and/or pump head failure.

The system 100 may include at least one pressure sensor 142 in communication with the fluid path, strategically positioned to monitor pressure levels within the tubing 120. In one exemplification, the at least one pressure sensor 142 may include an upstream pressure sensor 142a located around an upstream region 160 of the fluid path outside the tubing 120, configured to detect and measure at least one pressure value of the fluid passing through this area. It will be understood that the pressure sensors may be positioned elsewhere along the fluid circuit provided they are within a location where pressures in both upstream and downstream regions can be detected.

The upstream pressure sensor 142a is positioned to precisely sense the pressure of fluid entering the peristaltic pump 110, providing real-time data on the pressure conditions before the fluid reaches the pump's compression zone. This sensor is capable of detecting both static and dynamic pressure changes in the fluid path, allowing for continuous monitoring of fluid status as it flows through the upstream region 160.

By measuring pressure values in the upstream region 160, the sensor provides information for assessing the fluid's movement and flow rate, as well as identifying any potential issues such as blockages, leaks, or irregular fluid supply that could impact the overall system performance. The data from the upstream pressure sensor 142a can be used by the system's controller to adjust pumping parameters as needed, ensuring stable and efficient fluid conveyance through the tubing 120.

The system 100 may further include a controller configured to operate the peristaltic pumping unit 110 in a specific diagnostic mode. In this mode, the controller initiates the rotation of the rotor 152 while managing the position of the at least one roller 140 relative to the flexible tubing 120 to assess pressure values accurately. When the diagnostic mode is activated, the controller positions the roller 140 in an initial retracted position at time t=0, where the roller 140 is disengaged from the tubing 120, allowing fluid, such as saline solution, to flow freely in the tubing 120 and establish a baseline pressure in the system 100. In one system 100, the initial pressure value is taken before the roller 140 begins compressing the tubing 120 at time t=0, it represents the baseline pressure in the upstream region 160 before any compression occurs. If such baseline (or initial) pressure cannot be established, it may be an indication of any operational abnormality as discussed herein. Controller may be further configured to activate the peristaltic pumping unit 110 to allow for fluid flowing from the fluid source to the pumping unit 110, the roller 140 moving to the engaged position and rotating at least one roller within the pump race 130. When running the diagnostic mode in the system 100, saline solution can be used as substitute for the standard processing fluid. This substitution is particularly useful when the processing fluid is not readily available or its use is not feasible for non-operational tests. In addition, saline is used to prevent contamination or residue buildup. After a specified time interval Δt counting from t=0, the controller instructs the drive unit 150 to move the roller 140 to the engaged position. In this engaged position, the roller 140 compresses the tubing 120, partially or fully occluding it to create a localized increase in pressure. In another exemplary system 100, this compression allows the system 100 to measure another at least one pressure value (initial pressure value at upstream region 160) at the point of occlusion, which can be used to assess fluid dynamics, detect abnormalities in pressure levels, or monitor for potential leaks. In this exemplary system 100, only the case where the initial pressure value (whether or not it is taken from upstream region 160 or downstream region 180) of the at least one pressure value measure at time t=0 is described. However, it should be appreciated that the algorithm of measuring the initial pressure value as soon as the roller 140 starts compressing the tubing 120 after time interval Δt is equally applicable to other systems in determining the pressure gradient in upstream 160 and/or downstream regions 180.

The controller is further configured to perform the following steps to monitor and assess the operation of the peristaltic pump 110 based on pressure gradient developed from the upstream region 160:
(i) Receiving an Initial Upstream Pressure Value: The controller receives an upstream pressure value from the upstream pressure sensor 142a, which represents the pressure in the fluid passing through the upstream region 160 at an initial time t=0. This initial pressure reading serves as a baseline or reference pressure in the upstream region 160 before the roller 140 initiates its compressive action on the tubing 120.
(ii) Calculating a Pressure Difference Over Time: After the specified time interval Δt, the controller instructs the drive unit 150 to move the roller 140 to the engaged position. As the peristaltic pumping unit 110 operates, the controller monitors pressure values to assess changes in the upstream region 160. After the roller 140 completes at least one full rotation, the controller obtains another upstream pressure value from the upstream pressure sensor 142a, representing the pressure in the upstream region 160 after fluid has been moved by the pump's cyclical action. The controller then determines the difference between the initial upstream pressure value (at time t=0) and the subsequent upstream pressure value (after at least one cyclical rotation of the pump), reflecting any pressure variations due to the pump's operation.
(iii) Evaluating for Operational Abnormalities: By analyzing the pressure difference, the controller assesses whether the pressure gradient aligns with expected operational values. If the difference between the initial and subsequent upstream pressure values is outside a predefined range, this may indicate an operational anomaly, such as tubing 120 occlusion, leakage, roller malfunction, rotor failure, pump loop misloading, or other defect (such as, for example, a defect in the raceway).
(iv) Generating an Alert for Abnormal Conditions: If the controller's evaluation indicates a pressure deviation suggesting a pump operation abnormality, the controller generates an alert to notify the user or connected monitoring systems. This alert may be visual, auditory, or transmitted electronically to a remote monitoring device, allowing for timely intervention. The alert provides an early indication of potential issues, prompting inspection or maintenance to ensure safe and effective pump operation.

In another exemplified system 100, it may be configured to issue an alert if a measured pressure difference falls outside of a preset threshold range. This threshold is defined as a specific range that represents the acceptable pressure gradient between two measured upstream pressure values-one obtained before and the other obtained after at least one full rotation of the roller 140 in the peristaltic pump 110. The preset threshold value is designed to account for normal operational pressure fluctuations within the fluid system, distinguishing these from abnormal deviations that may indicate potential issues such as leakage, occlusion, or roller malfunction. When the pressure difference between the upstream pressure value at the start of the roller rotation and the subsequent upstream pressure value after the roller 140 completes at least one full rotation exceeds or falls below this preset threshold, the system 100 automatically generates an alert.

In yet another exemplary system 100 for pumping and distributing fluid, similar to the system in the previous aspect equipped with an upstream pressure sensor 142a but differing by the inclusion of a downstream pressure sensor 142b, the at least one pressure sensor 142 in this system 100 is configured to detect at least one pressure value of the fluid passing through the downstream region 180. In this exemplification, the at least one pressure sensor 142 may include the downstream pressure sensor 142b located around the downstream region 180 of the fluid path outside the tubing 120, configured to detect and measure at least one pressure value of the fluid passing through this area. By measuring downstream pressure, this sensor 142b provides real-time data on fluid conditions after the peristaltic pump's compression cycle, allowing the system to monitor for abnormalities such as unexpected pressure drops (indicative of leakage) or inconsistent pressure gradients (potentially signaling partial occlusions or pump head issues). When analyzed alongside upstream pressure readings, downstream pressure data enables the system to calculate accurate pressure gradients, facilitating precise monitoring of fluid flow and identifying operational irregularities.

In this exemplary system 100, the controller may be further configured to perform the additional steps as follows to monitor and assess the operation of the peristaltic pump 110 based on pressure gradient developed from the downstream region 180:
(v) Receiving an Initial Downstream Pressure Value: The controller receives a downstream pressure value from the downstream pressure sensor 142b, which represents the pressure in the fluid passing through the downstream region 180 at an initial time t=0. This initial pressure reading serves as a baseline or reference pressure in the downstream region 180 before the roller 140 initiates its compressive action on the tubing 120.
(vi) Calculating a Pressure Difference Over Time: As the peristaltic pumping unit 110 operates, the controller monitors pressure values to assess changes in the downstream region 180. After the roller 140 completes at least one full rotation, the controller obtains another downstream pressure value from the downstream pressure sensor 142b, representing the pressure in the downstream region 180 after fluid has been moved by the pump's cyclical action. The controller then determines the difference between the initial downstream pressure value (at time t=0) and the subsequent downstream pressure value (after at least one cyclical action), reflecting any pressure variations due to the pump's operation.
(vii) Evaluating for Operational Abnormalities: By analyzing the pressure difference, the controller assesses whether the pressure gradient aligns with expected operational values. If the difference between the initial and subsequent downstream pressure values is outside a predefined range, this may indicate an operational anomaly, such as tubing 120 occlusion, leakage, or roller malfunction.
(viii) Generating a Downstream Alert for Abnormal Conditions: If the controller's evaluation indicates a pressure deviation suggesting a pump operation abnormality, the controller generates a downstream alert to notify the user or connected monitoring systems. The downstream alert and the upstream alert designate the same pump operation abnormality. This alert may be visual, auditory, or transmitted electronically to a remote monitoring device, allowing for timely intervention. The alert provides an early indication of potential issues, prompting inspection or maintenance to ensure safe and effective pump operation.

In normal, leak-free operation, the pressure gradient falls within a specific range due to the regular cycle of compression and release by the roller 140. However, during abnormal pump operation, the pressure gradient in a peristaltic pump system 100 may develop differently depending on factors such as location of the leak (upstream 160 or downstream 180) or a pump head failure. For instance, regardless of the leak's location, the pressure gradient in the upstream region 160 might fall outside the expected range or, in some cases, no pressure may be generated at all. While the present disclosure emphasizes the pressure gradient in assessing operational abnormalities, it should be noted that pressure values measured from either region can also indicate the presence of abnormalities. For example, a leak in either the upstream or downstream region could result in a corresponding pressure value being close to or equal to atmospheric pressure. Additionally, the pressure values and pressure gradients for the upstream and downstream regions are independent. This means that the upstream and downstream pressure sensors will generate their own alerts independently, in response to specific pressure values measured and/or gradients computed, as the fluid dynamics in these regions are mutually exclusive. The specific details of how these abnormalities affect the pressure gradient are described as follows.

### 1. Leak in the Upstream Region

(i) Upstream pressure gradient: The pressure may fail to build up adequately because fluid escapes from the fluid circuit. This fluid escape limits the ability of the roller 140 to establish the expected pressure differential, resulting in a pressure difference that falls below the threshold. This weak or nearly absent pressure buildup is detected by the system 100 as an anomaly, leading to an alert signaling a possible upstream leak.
(ii) Downstream pressure gradient: The downstream region 180 may still experience some pressure, as the roller 140 continues to compress the tubing 120 after fluid is drawn from the inlet 122. However, because less fluid enters the system 100, the downstream pressure gradient may be lower than normal, reflecting a reduced volume of fluid moving through the pump 110.

### 2. Leak in the Downstream Region

(i) Upstream pressure gradient: If there is a leak in the downstream region 180, the upstream pressure would typically remain unaffected initially (as long as the tubing and roller mechanism are intact and occluding properly) and behave as expected under normal condition. However, in the situation where the system is operating with a peristaltic pump featuring a single roller, the leak over time can compromise the system's ability to maintain pressure separation. For example, if roller 140 fails to occlude the fluid flow, the upstream pressure may gradually decrease until it equalizes with the downstream pressure, causing the pressure gradient to drop to zero.
(ii) Downstream pressure gradient: The downstream region 180 loses pressure rapidly as fluid escapes through the leak, making it difficult to maintain normal pressure levels. As a result, the downstream pressure would not even be generated and the pressure gradient is likely to be very low or near zero, indicating that fluid is not effectively held within the tubing 120 past the pump head, leading to an alert signaling a possible downstream leak.

### 3. Pump Head Failure

(i) Upstream pressure gradient: When the pump head fails (e.g., the roller 140 does not the tubing 120 due to wear, misalignment or damage), the expected compression of the tubing 120 does not occur. This prevents the normal development of a pressure gradient in the upstream region 160, as there is minimal suction or compression occurring in the pump cycle. The upstream pressure gradient will therefore be very low or close to zero.
(ii) Downstream pressure gradient: Similarly, the downstream region 180 will not receive adequate pressure from the roller's compression, resulting in a diminished or nearly absent pressure gradient in the downstream region 180 as well..

In a scenario where both the upstream and downstream pressure gradients are diminished or nearly absent, this indicates minimal to no pressure change in both regions. This observation strongly suggests a lack of effective fluid movement within the pump 110, leading to the conclusion of pump head failure. The pump head failure alert may be even more precise in this case.

In another scenario where the system operates with a peristaltic pump equipped with multiple rollers, and assuming not all rollers are damaged or failed, the pressure gradient in both the upstream region 160 and downstream region 180 could still be established. However, the process may require a substantial amount of time. This delay could be detected by incorporating additional sensors and timers, or by using a smart pressure sensor having time-measurement capabilities.

The table below summarizes how the pressure gradients develop differently depending on the location of the leak (upstream 160 or downstream 180 region) or pump head failure in response to each of these situations:

**Table 1**

| **Condition** | **Upstream Pressure Gradient** | **Downstream Pressure Gradient** |
|---|---|---|
| **Leak in Upstream Region** | Low or near zero (due to fluid escape) | Lower than normal (reflecting reduced fluid flow) |
| **Leak in Downstream Region** | Refer to the aforementioned description for single roller situation and all other scenarios | Very low or zero (due to fluid escape) |
| **Pump Head Failure** | Low or zero (due to inadequate suction/compression) or significant delay in establishing gradient in multiple rollers pump system | Low or zero (due to ineffective compression) or significant delay in establishing gradient in multiple rollers pump system |

If the upstream and downstream regions are of similar size, or both regions are sufficiently small, the pressure changes caused by the peristaltic pump will be comparable in magnitude for each region. This similarity ensures that the pressure gradients developed in both regions should align with expected patterns under normal pump operation. The smaller the regions, the more localized the pressure changes, making the sensor readings more directly attributable to the pump's functionality and less influenced by external factors (e.g., tubing compliance or fluid dynamics over long distances).

In another example, the system 100 may be configured to issue an alert if the pressure difference between two measured upstream pressure values-one measured before the roller 140 completes at least one rotation (at t=0) and one measured after the roller 140 completes the rotation-is zero. This zero pressure difference may indicate that fluid is not moving as expected through the tubing 120, potentially due to a system malfunction, such as pump head failure, blockage, or another operational issue. Specifically, the zero pressure difference in the upstream region 160 may indicate the presence of a leak in the tubing 120, which disrupts normal pressure dynamics. In a leak-free system, the roller's movement through a full rotation cycle creates alternating high and low pressures. As the roller 140 compresses the tubing 120, it creates high pressure (positive pressure), forcing fluid downstream. After the roller 140 moves, the tubing 120 expands, creating low pressure (negative pressure), which draws more fluid into the pump 110. This cycle of compression and expansion repeats with each rotation of the roller 140, generating a continuous flow of fluid. The alternating high and low pressures at different points in the tube are responsible for driving the fluid through the pump 110, and developing pressure gradient along the tube over time.

The pressure gradient developed in the upstream and downstream regions is influenced by the size of the region, the number of rotor revolutions, and the stroke volume of the peristaltic pump. Larger regions allow for more gradual pressure changes, while smaller regions produce sharper gradients. A higher number of revolutions moves more fluid into the region, increasing the overall gradient over time. Similarly, a larger stroke volume displaces more fluid per cycle, resulting in stronger pressure gradients.

However, when a leak is present, fluid escapes from the tubing 120, preventing the expected pressure buildup. This escape of fluid causes the pressure to equalize quickly with the surrounding environment, resulting in a stable, nearly flat pressure reading in the upstream region 160.

As a result, the pressure sensor 142 detects no meaningful or nearly absent pressure gradient between before and after the roller 140 completes a rotation, indicating a possible pump head failure, where the roller 140 is not compressing the tubing effectively. This lack of fluctuation suggests that fluid is not being properly retained within the system 100, which can trigger an alert for maintenance to locate and replace the pump and/or pump head, thereby restoring normal operation.

In yet another example, the system 100 may include diagnostic capabilities to identify and categorize pump operation abnormalities, which may include tubing leakage or pump head failure. The development of an excessive or inadequate pressure gradient due to tubing leakage was discussed in the previous paragraphs and will not be repeated here. As far as pump head failure is concerned, such as a worn or damaged roller or drive unit malfunction, such failure compromises fluid flow by preventing the roller 140 from fully engaging with the tubing 120 or maintaining adequate pressure. If the pressure gradient is nearly absent (close to zero), it may indicate a pump head failure, where the roller is not compressing the tubing 120 effectively. In a normally functioning pump 110, the roller 140 generates alternating positive and negative pressures, creating a measurable pressure difference. However, if the roller 140 is worn or misaligned, or if the drive mechanism fails, the tubing 120 may not be adequately compressed, leading to a flat or negligible pressure gradient. The system 100 may interpret this as a potential pump head failure and issues an alert accordingly.

The determination of pressure gradients in the upstream and downstream regions is equally applicable when the peristaltic pump operates in the opposite direction, as shown in Fig. 2. In this scenario, the roles of the pressure sensors 142a, 142b and the upstream and downstream regions are reversed (i.e. the downstream region is designated as "160", the upstream region is designated as "180", the downstream pressure senor is designated as "142a" and the upstream pressure sensor is designated as "142b"), with the pressure gradients reflecting the reversed flow of fluid through the system. Otherwise, the configuration of the system illustrated in Fig. 2 is substantially the same as that of the system shown in Fig. 1, as described above, and will not be repeated here for brevity.

### Aspects

Aspect 1. A system for pumping and distributing fluid comprising flexible tubing defining a fluid path for conveying biological or medical fluid from a source for processing, an external peristaltic pumping unit associated with the flexible tubing, said peristaltic pumping unit comprising a pump race for receiving the tubing, a pump rotor carrying at least one roller, and a drive unit for rotating the rotor and thereby rotating the at least one roller within the pump race; wherein the flexible tubing having an upstream region positioned before the roller and a downstream region positioned after the roller; at least one pressure sensor in communication with the fluid path, wherein the at least one pressure sensor includes an upstream pressure sensor configured to detect at least one pressure value of the fluid passing through the upstream region; a controller configured to operate the peristaltic pumping unit in a diagnostic mode, in which the rotor is rotated with the at least one roller initially positioned away from the tubing at time t=0; and the drive unit drives the at least one roller to engage the tubing after a time interval Δt, thereby compressing the tubing to measure the at least one pressure value, wherein the controller is further configured to receive from the upstream pressure sensor a first upstream pressure value of the at least one pressure value of the pressure in the fluid passing through the upstream region at time t=0; determine a difference between the first upstream pressure value and a second upstream pressure value, wherein the second upstream pressure value corresponds to the pressure in the fluid passing through the upstream region obtained from the upstream pressure sensor after the at least one roller completes at least one rotation; and generate an upstream alert if the determination indicates pump operation abnormality.

Aspect 2. The system for pumping and distributing fluid of aspect 1, wherein the upstream alert is issued if the difference exceeds or falls below a preset upstream threshold range, the preset upstream threshold range corresponding to an expected upstream pressure gradient between upstream pressure values measured before and after the at least one roller completes a rotation.

Aspect 3. The system for pumping and distributing fluid of aspect 1, wherein the upstream alert is generated if the difference is zero or nearly absent.

Aspect 4. The system of aspect 1, wherein the upstream alert is generated if the first upstream pressure value is close to or equal to atmospheric pressure.

Aspect 5. The system of aspect 1, wherein the upstream alert is generated if the second upstream pressure value is close to or equal to atmospheric pressure.

Aspect 6. The system for pumping and distributing fluid of aspect 1, wherein the pump operation abnormality comprises a tubing leakage or a pump head failure.

Aspect 7. The system for pumping and distributing fluid of aspect 1, wherein the at least one pressure sensor further includes a downstream pressure sensor configured to detect at least one pressure value of the fluid passing through the downstream region.

Aspect 8. The system for pumping and distributing fluid of aspect 7, wherein the controller is further configured to, receive from the downstream pressure sensor a first downstream pressure value of the at least one pressure value of the pressure in the fluid passing through the downstream region at time t=0; determine a difference between the first downstream pressure value and a second downstream pressure value, wherein the second downstream pressure value corresponds to the pressure in the fluid passing through the downstream region obtained from the downstream pressure sensor after the at least one roller completes at least one rotation; and generate a downstream alert if the determination indicates the pump operation abnormality, wherein the upstream alert and the downstream alert designating the same abnormality.

Aspect 9. The system for pumping and distributing fluid of aspect 8, wherein the downstream alert is issued if the difference exceeds or falls below a preset downstream threshold range, the preset downstream threshold range corresponding to an expected downstream pressure gradient between downstream pressure values measured before and after the at least one roller completes a rotation.

Aspect 10. The system for pumping and distributing fluid of aspect 8, wherein the downstream alert is issued if the difference is nearly absent or zero.

Aspect 11. A system for pumping and distributing fluid comprises flexible tubing defining a fluid path for conveying biological or medical fluid from a source for processing, an external peristaltic pumping unit associated with the flexible tubing, said peristaltic pumping unit comprising a pump race for receiving the tubing, a pump rotor carrying at least one roller, and a drive unit for rotating the rotor and thereby rotating the at least one roller within the pump race; wherein the flexible tubing having an upstream region positioned before the roller and a downstream region positioned after the roller; at least one pressure sensor in communication with the fluid path, wherein the at least one pressure sensor includes a downstream pressure sensor configured to detect at least one pressure value of the fluid passing through the downstream region; a controller configured to operate the peristaltic pumping unit in a diagnostic mode, in which the rotor is rotated with the at least one roller initially positioned away from the tubing at time t=0; and the drive unit which drives the at least one roller to engage the tubing after a time interval Δt, thereby compressing the tubing to measure the at least one pressure value, wherein the controller is further configured to, receive from the downstream pressure sensor a first downstream pressure value of the at least one pressure value of the pressure in the fluid passing through the downstream region at time t=0; determine a difference between the first downstream pressure value and a second downstream pressure value, wherein the second downstream pressure value corresponds to the pressure in the fluid passing through the downstream region obtained from the downstream pressure sensor after the at least one roller completes at least one rotation; and generate a downstream alert if the determination indicates pump operation abnormality.

Aspect 12. The system for pumping and distributing fluid of aspect 11, wherein the pump operation abnormality comprises a tubing leakage or a pump head failure.

Aspect 13. The system for pumping and distributing fluid of aspect 11, wherein the downstream alert is issued if the difference exceeds or falls below a preset downstream threshold range, the preset downstream threshold range corresponding to an expected downstream pressure gradient between downstream pressure values measured before and after the at least one roller completes a rotation.

Aspect 14. The system for pumping and distributing fluid of aspect 11, wherein the downstream alert is issued if the difference is nearly absent or zero.

Aspect 15. The system of claim 11, wherein the downstream alert is generated if the first downstream pressure value is close to or equal to atmospheric pressure.

Aspect 16. The system of claim 11, wherein the downstream alert is generated if the second downstream pressure value is close to or equal to atmospheric pressure.

Aspect 17. A method for identifying a malfunction in a tubing kit mounted on a peristaltic pump unit for pumping and distributing fluid, the peristaltic pumping unit comprising at least one roller, a flexible tubing having an upstream region positioned before the at least one roller where fluid enters the pump and a downstream region positioned after the at least one roller where fluid exits the pump, a race configured to receive the tubing, and at least one pressure sensor, the method comprising steps: (i) placing a tubing in flow communication with fluid source onto the peristaltic pumping element included in the system; (ii) activating the peristaltic pumping unit with a diagnostic mode to allow the at least one roller moving to a retracted position in which the at least one roller is positioned away from the race; loading the tubing to the race; (iii) activating the peristaltic pumping unit to allow for fluid flowing from the fluid source to the pumping unit, the at least one roller moving to an engaged position in which the at least one roller engages with the race and rotating the at least one roller within the pump race; (iv) obtaining from an upstream pressure sensor included in the at least one pressure sensor a first upstream pressure value corresponding to the pressure in the fluid passing through an upstream region at time t=0; (v) allowing the at least one roller to complete at least one rotation, wherein the at least one roller moving to the retracted position after completing the at least one rotation; (vi) obtaining from the upstream pressure sensor a second upstream pressure value corresponding to the pressure in the fluid passing through the upstream region after step (v); (vii) determining an upstream pressure difference between the first upstream pressure value and the second upstream pressure value; (viii) generating a first alert if the upstream pressure difference exceeds or falls below a preset threshold range, wherein the preset threshold range corresponding to an expected upstream pressure gradient between upstream pressure values measured before and after the at least one roller completes a rotation; or (ix) generating a second alert if the upstream pressure difference is zero or nearly absent.

Aspect 18. The method for identifying a malfunction in a tubing kit mounted on a peristaltic pump for pumping and distributing fluid according to aspect 17, further comprises steps: after step (iv) and before step (v), obtaining from a downstream pressure sensor included in the at least one pressure sensor a first downstream pressure value corresponding to the pressure in the fluid passing through the downstream region at time t=0; after step (vi) and before step (vii), obtaining from the downstream pressure sensor a second downstream pressure value corresponding to the pressure in the fluid passing through the downstream region; determining a downstream pressure difference between the first downstream pressure value and the second downstream pressure value; generating a third alert if the second pressure difference is zero or nearly absent.

Aspect 19. The method for identifying a malfunction in a tubing kit mounted on a peristaltic pump for pumping and distributing fluid according to claim 17 or 18, further comprising generating a fourth alert if the second upstream pressure value is close to or equal to atmospheric pressure.

Aspect 20. The method for identifying a malfunction in a tubing kit mounted on a peristaltic pump for pumping and distributing fluid according to aspect 18, further comprising generating a fifth alert if the second downstream pressure value is close to or equal to atmospheric pressure.

Aspect 21. A method for identifying a malfunction in a tubing kit mounted on a peristaltic pump unit for pumping and distributing fluid, the peristaltic pumping unit comprising at least one roller, a flexible tubing having an upstream region positioned before the at least one roller where fluid enters the pump and a downstream region positioned after the at least one roller where fluid exits the pump, a race configured to receive the tubing, and at least one pressure sensor, the method comprising steps: (i) placing a tubing in flow communication with fluid source onto the peristaltic pumping element included in the system; (ii) activating the peristaltic pumping unit with a diagnostic mode to allow the at least one roller moving to a retracted position in which the at least one roller is positioned away from the race; (iii) activating the peristaltic pumping unit to allow for fluid flowing from the fluid source to the pumping unit, the at least one roller moving to an engaged position in which the at least one roller engages with the race and rotating at least one roller within the pump race; (iv) obtaining from a downstream pressure sensor included in the at least one pressure sensor a first downstream pressure value corresponding to the pressure in the fluid passing through the downstream region at time t=0; (v) allowing the at least one roller to complete at least one rotation, wherein the at least one roller moving to the retracted position after completing the at least one rotation; (vi) obtaining from the downstream pressure sensor a second upstream pressure value corresponding to the pressure in the fluid passing through the downstream region after step (v); (vii) determining a downstream pressure difference between the first downstream pressure value and the second downstream pressure value; (viii) generating a first alert if the downstream pressure difference exceeds or falls below a preset threshold range, wherein the preset threshold range corresponding to an expected downstream pressure gradient between downstream pressure values measured before and after the at least one roller completes a rotation; or (ix) generating a second alert if the downstream pressure difference is zero or nearly absent.

Aspect 22. The method for identifying a malfunction in a tubing kit mounted on a peristaltic pump for pumping and distributing fluid according to aspect 21, further comprising generating a third alert if the second downstream pressure value is close to or equal to atmospheric pressure.

Aspect 23. A method for identifying pump head failure in a system for pumping and distributing fluid including peristaltic pumping element comprising at least one roller, a flexible tubing having an upstream region positioned before the at least one roller where fluid enters the pump and a downstream region positioned after the at least one roller where fluid exits the pump, a race configured to receive the tubing, and at least one pressure sensor, wherein the method comprising steps: (i) placing a tubing in flow communication with fluid source onto the peristaltic pumping element included in the system; (ii) activating the peristaltic pumping element with a diagnostic mode to allow the at least one roller moving to a retracted position in which the at least one roller is positioned away from the race; (iii) activating the peristaltic pumping element to allow for fluid flowing from the fluid source to the pumping unit, the at least one roller moving to an extended position in which the at least one roller engages with the race and rotating the at least one roller within the pump race ; (iv) obtaining from a upstream pressure sensor included in the at least one pressure sensor a first upstream pressure value corresponding to the pressure in the fluid passing through the upstream region at time t=0; (v) allowing the at least one roller to complete at least one rotation in the diagnostic mode, wherein the at least one roller moving to the retracted position after completing the at least one rotation; (vi) obtaining from the upstream pressure sensor a second upstream pressure value corresponding to the pressure in the fluid passing through the upstream region after step (v); (vii) determining upstream pressure difference between the first upstream pressure value and the second upstream pressure value; (viii) generating a pump head failure alert if the upstream pressure difference is zero or nearly absent.

Aspect 24. The method for identifying a pump head failure in a system for pumping and distributing fluid according to aspect 23, further comprising: after step (iv) and before step (v), obtaining from a downstream pressure sensor a first downstream pressure value corresponding to the pressure in the fluid passing through the downstream region at time t=0; after step (vi) and before step (vii), obtaining from the downstream pressure sensor a second downstream pressure value corresponding to the pressure in the fluid passing through the downstream region; determining a downstream pressure difference between the first downstream pressure value and the second downstream pressure value; generating a precise pump head failure alert if both the upstream pressure difference and the downstream pressure difference are zero or nearly absent.

Aspect 25. A method for identifying a pump head failure in a system for pumping and distributing fluid including peristaltic pumping element comprising at least one roller, a flexible tubing having an upstream region positioned before the at least one roller where fluid enters the pump and a downstream region positioned after the at least one roller where fluid exits the pump, a race configured to receive the tubing, and at least one pressure sensor, wherein the method comprising steps: (i) placing a tubing in flow communication with fluid source onto the peristaltic pumping element included in the system; (ii) activating the peristaltic pumping element with a diagnostic mode to allow the at least one roller moving to a retracted position in which the at least one roller is positioned away from the race; (iii) activating the peristaltic pumping element to allow for fluid flowing from the fluid source to the pumping unit, at least one roller moving to an extended position in which at least one roller engages with the race and rotating at least one roller within the pump race; (iv) obtaining from a downstream pressure sensor included in the at least one pressure sensor a first downstream pressure value corresponding to the pressure in the fluid passing through the downstream region at time t=0; (v) allowing at least one roller to complete at least one rotation in the diagnostic mode; (vi) obtaining from the downstream pressure sensor a second downstream pressure value corresponding to the pressure in the fluid passing through the downstream region after step (v); (vii) determining the downstream pressure difference between the first downstream pressure value and the second downstream pressure value; (viii) generating a pump head failure alert if the downstream pressure difference is zero or nearly absent.

## Claims

1. A system for pumping and distributing fluid comprising:
flexible tubing defining a fluid path for conveying biological or medical fluid from a source for processing,
an external peristaltic pumping unit associated with the flexible tubing, said peristaltic pumping unit comprising a pump race for receiving the tubing, a pump rotor carrying at least one roller, and a drive unit for rotating the rotor and thereby rotating the at least one roller within the pump race;
wherein the flexible tubing having an upstream region positioned before the roller and a downstream region positioned after the roller;
at least one pressure sensor in communication with the fluid path, wherein the at least one pressure sensor includes an upstream pressure sensor configured to detect at least one pressure value of the fluid passing through the upstream region;
a controller configured to operate the peristaltic pumping unit in a diagnostic mode, in which the rotor is rotated with the at least one roller initially positioned away from the tubing at time t=0; and the drive unit drives the at least one roller to engage the tubing after a time interval Δt, thereby compressing the tubing to measure the at least one pressure value,
wherein the controller is further configured to,
receive from the upstream pressure sensor a first upstream pressure value of the at least one pressure value of the pressure in the fluid passing through the upstream region at time t=0;
determine a difference between the first upstream pressure value and a second upstream pressure value, wherein the second upstream pressure value corresponds to the pressure in the fluid passing through the upstream region obtained from the upstream pressure sensor after the at least one roller completes at least one rotation; and
generate an upstream alert if the determination indicates pump operation abnormality.

2. The system of claim 1, wherein the upstream alert
- is issued if the difference exceeds or falls below a preset upstream threshold range, the preset upstream threshold range corresponding to an expected upstream pressure gradient between upstream pressure values measured before and after the at least one roller completes a rotation, and/or
- is generated if the difference is zero or nearly absent, and/or
- is generated if the first upstream pressure value is close to or equal to atmospheric pressure, and/or
- is generated if the second upstream pressure value is close to or equal to atmospheric pressure.

3. The system of claim 1, wherein the pump operation abnormality comprises a tubing leakage or a pump head failure.

4. The system of claim 1, wherein the at least one pressure sensor further includes a downstream pressure sensor configured to detect at least one pressure value of the fluid passing through the downstream region.

5. A system for pumping and distributing fluid comprising:
flexible tubing defining a fluid path for conveying biological or medical fluid from a source for processing,
an external peristaltic pumping unit associated with the flexible tubing, said peristaltic pumping unit comprising a pump race for receiving the tubing, a pump rotor carrying at least one roller, and a drive unit for rotating the rotor and thereby rotating the at least one roller within the pump race;
wherein the flexible tubing having an upstream region positioned before the roller and a downstream region positioned after the roller;
at least one pressure sensor in communication with the fluid path, wherein the at least one pressure sensor includes a downstream pressure sensor configured to detect at least one pressure value of the fluid passing through the downstream region;
a controller configured to operate the peristaltic pumping unit in a diagnostic mode, in which the rotor is rotated with the at least one roller initially positioned away from the tubing at time t=0; and the drive unit drives the at least one roller to engage the tube after a time interval Δt, thereby compressing the tubing to measure the at least one pressure value,
wherein the controller is further configured to,
receive from the downstream pressure sensor a first downstream pressure value of the at least one pressure value of the pressure in the fluid passing through the downstream region at time t=0;
determine a difference between the first downstream pressure value and a second downstream pressure value, wherein the second downstream pressure value corresponds to the pressure in the fluid passing through the downstream region obtained from the downstream pressure sensor after the at least one roller completes at least one rotation; and
generate a downstream alert if the determination indicates pump operation abnormality.

6. The system of claim 5, wherein the pump operation abnormality comprises a tubing leakage or a pump head failure.

7. The system of claim 5, wherein the downstream alert
- is issued if the difference exceeds or falls below a preset downstream threshold range, the preset downstream threshold range corresponding to an expected downstream pressure gradient between downstream pressure values measured before and after the at least one roller completes a rotation, and/or
- is issued if the difference is nearly absent or zero, and/or
- is generated if the first downstream pressure value is close to or equal to atmospheric pressure, and/or
- is generated if the second downstream pressure value is close to or equal to atmospheric pressure.

8. A method for identifying a malfunction in a tubing kit mounted on a peristaltic pump unit for pumping and distributing fluid, the peristaltic pumping unit comprising at least one roller, a flexible tubing having an upstream region positioned before the at least one roller where fluid enters the pump and a downstream region positioned after the at least one roller where fluid exits the pump, a race configured to receive the tubing, and at least one pressure sensor, the method comprising steps:
(i) placing a tubing in flow communication with fluid source onto the peristaltic pumping element included in the system;
(ii) activating the peristaltic pumping unit with a diagnostic mode to allow the at least one roller moving to a retracted position in which the at least one roller is positioned away from the race;
(iii) activating the peristaltic pumping unit to allow for fluid flowing from the fluid source to the pumping unit, the at least one roller moving to an engaged position in which the at least one roller engages with the race and rotating the at least one roller within the pump race;
(iv) obtaining from an upstream pressure sensor included in the at least one pressure sensor a first upstream pressure value corresponding to the pressure in the fluid passing through an upstream region at time t=0;
(v) allowing the at least one roller to complete at least one rotation, wherein the at least one roller moving to the retracted position after completing the at least one rotation;
(vi) obtaining from the upstream pressure sensor a second upstream pressure value corresponding to the pressure in the fluid passing through the upstream region after step (v);
(vii) determining an upstream pressure difference between the first upstream pressure value and the second upstream pressure value;
(viii) generating a first alert if the upstream pressure difference exceeds or falls below a preset threshold range, wherein the preset threshold range corresponding to an expected upstream pressure gradient between upstream pressure values measured before and after the at least one roller completes a rotation; or
(ix) generating a second alert if the upstream pressure difference is zero or nearly absent.

9. The method for identifying a malfunction in a tubing kit mounted on a peristaltic pump for pumping and distributing fluid according to claim 8, further comprising:
after step (iv) and before step (v), obtaining from a downstream pressure sensor included in the at least one pressure sensor a first downstream pressure value corresponding to the pressure in the fluid passing through the downstream region at time t=0;
after step (vi) and before step (vii), obtaining from the downstream pressure sensor a second downstream pressure value corresponding to the pressure in the fluid passing through the downstream region;
determining a downstream pressure difference between the first downstream pressure value and the second downstream pressure value;
generating a third alert if the second pressure difference is zero or nearly absent.

10. The method for identifying a malfunction in a tubing kit mounted on a peristaltic pump for pumping and distributing fluid according to claim 8 or 9, further comprising generating a fourth alert if the second upstream pressure value is close to or equal to atmospheric pressure.

11. A method for identifying a malfunction in a tubing kit mounted on a peristaltic pump unit for pumping and distributing fluid, the peristaltic pumping unit comprising at least one roller, a flexible tubing having an upstream region positioned before the at least one roller where fluid enters the pump and a downstream region positioned after the at least one roller where fluid exits the pump, a race configured to receive the tubing, and at least one pressure sensor, the method comprising steps:
(i) placing a tubing in flow communication with fluid source onto the peristaltic pumping element included in the system;
(ii) activating the peristaltic pumping unit with a diagnostic mode to allow the at least one roller moving to a retracted position in which the at least one roller is positioned away from the race;
(iii) activating the peristaltic pumping unit to allow for fluid flowing from the fluid source to the pumping unit, the at least one roller moving to an engaged position in which the at least one roller engages with the race and rotating at least one roller within the pump race;
(iv) obtaining from a downstream pressure sensor included in the at least one pressure sensor a first downstream pressure value corresponding to the pressure in the fluid passing through the downstream region at time t=0;
(v) allowing the at least one roller to complete at least one rotation, wherein the at least one roller moving to the retracted position after completing the at least one rotation;
(vi) obtaining from the downstream pressure sensor a second upstream pressure value corresponding to the pressure in the fluid passing through the downstream region after step (v);
(vii) determining a downstream pressure difference between the first downstream pressure value and the second downstream pressure value;
(viii) generating a first alert if the downstream pressure difference exceeds or falls below a preset threshold range, wherein the preset threshold range corresponding to an expected downstream pressure gradient between downstream pressure values measured before and after the at least one roller completes a rotation; or
(ix) generating a second alert if the downstream pressure difference is zero or nearly absent.

12. The method for identifying a malfunction in a tubing kit mounted on a peristaltic pump for pumping and distributing fluid according to claim 11, further comprising generating a third alert if the second downstream pressure value is close to or equal to atmospheric pressure.

13. A method for identifying a pump head failure in a system for pumping and distributing fluid including peristaltic pumping element comprising at least one roller, a flexible tubing having an upstream region positioned before the at least one roller where fluid enters the pump and a downstream region positioned after the at least one roller where fluid exits the pump, a race configured to receive the tubing, and at least one pressure sensor, wherein the method comprising steps:
(i) placing a tubing in flow communication with fluid source onto the peristaltic pumping element included in the system;
(ii) activating the peristaltic pumping element with a diagnostic mode to allow the at least one roller moving to a retracted position in which the at least one roller is positioned away from the race;
(iii) activating the peristaltic pumping element to allow for fluid flowing from the fluid source to the pumping unit, the at least one roller moving to an extended position in which the at least one roller engages with the race and rotating the at least one roller within the pump race;
(iv) obtaining from an upstream pressure sensor included in the at least one pressure sensor a first upstream pressure value corresponding to the pressure in the fluid passing through the upstream region at time t=0;
(v) allowing the at least one roller to complete at least one rotation in the diagnostic mode, wherein the at least one roller moving to the retracted position after completing the at least one rotation;
(vi) obtaining from the upstream pressure sensor a second upstream pressure value corresponding to the pressure in the fluid passing through the upstream region after step (v);
(vii) determining upstream pressure difference between the first upstream pressure value and the second upstream pressure value;
(viii) generating a pump head failure alert if the upstream pressure difference is zero or nearly absent.

14. The method for identifying a pump head failure in a system for pumping and distributing fluid according to claim 13, further comprising:
after step (iv) and before step (v), obtaining from a downstream pressure sensor a first downstream pressure value corresponding to the pressure in the fluid passing through the downstream region at time t=0;
after step (vi) and before step (vii), obtaining from the downstream pressure sensor a second downstream pressure value corresponding to the pressure in the fluid passing through the downstream region;
determining a downstream pressure difference between the first downstream pressure value and the second downstream pressure value;
generating a precise pump head failure alert if both the upstream pressure difference and the downstream pressure difference are zero or nearly absent.

15. A method for identifying a pump head failure in a system for pumping and distributing fluid including peristaltic pumping element comprising at least one roller, a flexible tubing having an upstream region positioned before the at least one roller where fluid enters the pump and a downstream region positioned after the at least one roller where fluid exits the pump, a race configured to receive the tubing, and at least one pressure sensor, wherein the method comprising steps:
(i) placing a tubing in flow communication with fluid source onto the peristaltic pumping element included in the system;
(ii) activating the peristaltic pumping element with a diagnostic mode to allow the at least one roller moving to a retracted position in which the at least one roller is positioned away from the race;
(iii) activating the peristaltic pumping element to allow for fluid flowing from the fluid source to the pumping unit, at least one roller moving to an extended position in which at least one roller engages with the race and rotating at least one roller within the pump race;
(iv) obtaining from a downstream pressure sensor included in the at least one pressure sensor a first downstream pressure value corresponding to the pressure in the fluid passing through the downstream region at time t=0;
(v) allowing at least one roller to complete at least one rotation in the diagnostic mode;
(vi) obtaining from the downstream pressure sensor a second downstream pressure value corresponding to the pressure in the fluid passing through the downstream region after step (v);
(vii) determining the downstream pressure difference between the first downstream pressure value and the second downstream pressure value;
(viii) generating a pump head failure alert if the downstream pressure difference is zero or nearly absent.
